(19) Europäisches Patentamt
European Patent Office
Office européen des brevets

(11) Numéro de publication: **0 223 647 B1**

(12) **FASCICULE DE BREVET EUROPEEN**

(45) Date de publication du fascicule du brevet: **01.08.90**

(51) Int. Cl.$^5$: **C 07 D 213/40,** C 07 D 213/89, A 61 K 31/44

(21) Numéro de dépôt: **86402210.8**

(22) Date de dépôt: **06.10.86**

(54) **Alkylcarboxamides de pyridylalkylamines, leurs préparations et leur utilisation en tant que médicaments.**

(30) Priorité: **11.10.85 MA 20776**

(43) Date de publication de la demande: **27.05.87 Bulletin 87/22**

(45) Mention de la délivrance du brevet: **01.08.90 Bulletin 90/31**

(84) Etats contractants désignés: **AT BE CH DE ES GB GR IT LI LU NL SE**

(56) Documents cités:
**GB-A-2 048 248**
**US-A-3 951 995**

(73) Titulaire: **S.A. PANMEDICA Société dite: Zone Industrielle - Ilot J F-06510 Carros (FR)**

(72) Inventeur: **Laruelle, Claude Avenue Bellevue F-06270 Villeneuve Loubet (FR)**
Inventeur: **Lepant, Marcel 7, rue Mellarède F-06100 Nice (FR)**
Inventeur: **Raynier, Bernard 9, Chemin du Malvan F-06800 Cagnes (FR)**

(74) Mandataire: **Orès, Bernard et al Cabinet ORES 6, Avenue de Messine F-75008 Paris (FR)**

Il est rappelé que: Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition. (Art. 99(1) Convention sur le brevet européen).

EP 0 223 647 B1

Courier Press, Leamington Spa, England.

**Description**

La présente invention concerne de nouveaux amides aliphatiques et cycloaliphatiques des pyridylalkylamines ou de leurs dérivés N-oxyde de formule générale:

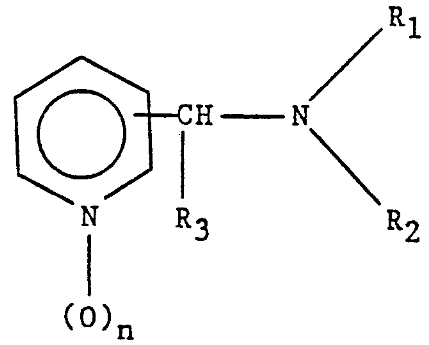

(I)

dans laquelle:

n est égal à 1 ou à 0,

$R_3$ représente un groupement alkyl de $C_1$ à $C_3$ ou un atome d'hydrogène,

$R_1$ représente un atome d'hydrogène,

$R_2$ représente un groupement:

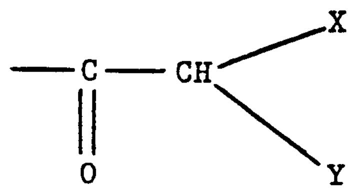

(II)

dans lequel:

X et Y sont différents,

X et Y représentent chacun un enchainement hydrocarboné saturé ou insaturé linéaire ou ramifié comprenant de 1 à 16 atomes de carbone.

X et Y déterminent ensemble un radical cyclique saturé ou insaturé comprenant de 3 à 12 atomes de carbone.

Les composés selon la présente invention présentent des propriétés pharmacologiques particulièrement intéressantes à savoir, para sympathomimétiques, antihypertensives, bradycardisantes, antianoxiques, analgésiques, antispasmodiques et broncholytiques.

SANOFI dans son brevet E.P. 101.380 décrivait l'amide nicotinique de la picolylamine. Plus tard, RIKER dans US 4.452.983 revendiquait le trifluoro-2 éthoxy-2 Hydroxy-5 benzamide picolinique.

BENOIT-GUYOD dans Chimie Thérapeutique (1968) — 336 étudiait les activités des amides de l'acide dipropylacétique et notait l'absence de propriétés anticonvulsivantes de la série.

MADE dans US 3 951 985 décrit des dipropylacétamides N substitués considérés particuliérement comme anticonvulsivants.

La Demanderesse a découvert de façon surprenante que les amides selon la formule générale I possédaient les activités pharmacologiques citées précédemment et pouvaient constituer des médicaments particuliérement intéressants.

La présente invention concerne également un procédé de préparation des dérivés de formule générale I. Ledit procédé est caractérisé en ce que l'on fait reàgir

un dérivé de formule générale III:

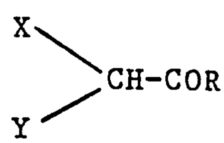

(III)

dans laquelle

X et Y sont tels que définis ci-dessus, et

R représente

un atome d'halogène,

un groupe

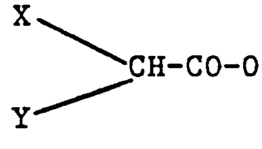

un groupe OH,
un groupe

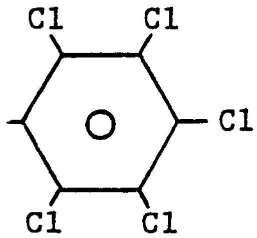

un groupe $(CH_3)_3C—CO—O$, ou
un groupe $C_2H_5—O—CO—O$, et
une pyridinealkylamine en quantité équimoléculaire, en solution dans un solvant inerte approprié, éventuellement en présence d'un accepteur d'hydrogène notamment une amine tertiaire choisie notamment dans le groupe qui comprend la triéthylamine, la pyridine, la picoline, à une température comprise entre la température ambiante et la température de reflux du milieu réactionnel.

Ce dérivé de formule III peut être un halogénure (R = halogène) ou un anhydride d'acide, un ester réactif ou un anhydride mixte.

Parmi les halogénures d'acide on utilise de préférence le chlorure qui est commercial ou peut être préparé de façon habituelle. Dans ce cas, la réaction est normalement réalisée avec la pyridylméthanamine dans un solvant inerte tel que le benzène, le toluène, l'éther ou un halogénoalcane.

On utilise un accepteur d'hydrogène qui peut être une amine tertiaire comme la triéthylamine, la pyridine, la picoline ou encore un excès de la pyridylméthanamine qui est ensuite récupérée à l'état de chlorhydrate. Les températures de réaction peuvent être comprises entre la température ordinaire et la température de reflux du milieu réactionnel. Les températures de réaction les plus élevées peuvent être employées avec les dérivés de formule (III) les moins réactifs.

D'autres dérivés de formule générale III peuvent être utilisés comme des esters tels que le pentachlorophényl ester, auquel cas on opère à température ambiante et dans un solvant polaire inerte tel que le diméthylformamide de préférence à température ordinaire.

On peut également utiliser un anhydride mixte comme dérivé réactif de formule générale (III) réalisé par exemple avec le chlorure de pivaloyle ou le chloroformate d'éthyle en présence d'une amine tertiaire comme la triéthylamine en solution dans l'acétone.

On peut encore faire réagir l'acide carboxylique de formule générale III (R = OH) sur la pyridineméthanamine en présence d'un catalyseur déshydratant comme une carbodiimide par exemple la dicyclo-hexylcarbodiimide en solution dans le diméthylformamide ou dans un solvant chloré inerte, à température ordinaire.

Selon une modalité particulière de la présente invention, on peut effectuer la réaction du chlorure d'acide de formule III (R = Cl) en solution dans le chloroforme avec la quantité équimoléculaire de pyridineméthanamine. L'amide basique formé restant en solution dans le solvant sert alors d'accepteur d'acide chlorhydrique et l'on peut récupérer de la solution chloroformique, après filtration de traces de chlorure de pyridineméthanamine insoluble, les dérivés de la présente invention.

On peut également reprendre à l'eau le résidu d'évaporation du chloroforme et obtenir la base à l'état pur aprés alcalinisation par une base minérale. Les amides ainsi isolés peuvent être salifiés par des acides minéraux ou organiques pharmaceutiquement acceptables.

Les dérivés N-oxyde de formule générale I peuvent être préparés à partir des amides de la pyridineméthanamine par action de l'eau oxygénée en solution dans l'acide acétique.

La température du milieu réactionnel peut être réglée entre la température ordinaire et la température du reflux de préférence entre 60° et 100°C.

L'isolement des dérivés N-oxyde recherchés se fait par évaporation de l'acide acétique sous pression réduite, extraction par un solvant non miscible à l'eau, lavage à la soude diluée et évaporation.

La reprise de l'évaporat par l'éther aboutit aux dérivés N-oxyde de la présente demande à l'état pur. Les produits selon l'invention sont purs en chromatographie sur couche mince dans les systèmes solvants indiqués et leur analyse élémentaire correspond aux résultats théoriques calculés.

La présente demande concerne également une composition pharmaceutique comprenant au moins un dérivé de formule générale (I) à l'état de base ou d'un de ses sels pharmaceutiquement acceptables. Ces compositions peuvent être préparées dans le but d'une administration orale ou parentérale avec les adjuvants habituellement employés. L'administration peut également être prévue par suppositoire ou préparation percutanée avec les vecteurs convenablement choisis.

ETUDE PHARMACOLOGIQUE

1) Potentialisation de l'action hypnotique du pentobarbital

Les dérivés selon l'invention sont injectés par voie intrapéritonéale à des souris, puis après 30 mn, on

administre 50 mg/kg i.p. de pentobarbital. On note une potentialisation très significative de la narcose pour des doses comprises entre 6 et 24 mg/kg.

2) Activité analgésique

Par administration à la souris des dérivés à la dose de 6, 12, 24 mg/kg, on note dans le test à l'acide acétique une activité analgésique significative dès 12 mg/kg.

Dans le test à la plaque chauffante, cette activité est notée dès 6 mg/kg par voie intrapéritonéale et à 24 mg/kg par voie orale.

On ne note pas de répose analgésique vis-à-vis de la stimulation électrique aux doses employées.

3) Activité antispasmodique

Testés sur le jéjunum de lapin et sur l'iléum de cobaye, les dérivés selon l'invention exercent un effet antagoniste vis-à-vis de l'histamine variant de 10 à 50% pour des concentrations dans le milieu de 1 à 2 × $10^{-2}$ µg/ml.

L'action spasmolytique est confirmée pour certains dérivés sur l'utérus isolé.

4) Activité bronchodilatatrice

On note un effet protecteur vis-à-vis du bronchospasme à l'histamine sur l'animal anesthésié dès la dose de 20 mg/kg pour certains dérivés de la présente demande.

Dans le spasme à l'acétylcholine, le dérivé de l'exemple 7 administré à 50 mg/kg p.o. exerce une protection bronchique de 100%.

5) Activité antianoxique

Les dérivés selon l'invention protègent également contre l'hypoxie expérimentale chez la souris.

Dans l'anoxie au curare (Flaxedyl à 16 mg/kg), on note un retard de la crise convulsive de 30 à 50% notamment avec les dérivés des exemples 5, 7, 11, 16, après administration intrapéritonéale à la dose de 100 mg/kg. L'effet anticurarisant est suffisant pour que certains animaux survivent à la crise convulsive.

Dans la test d'anoxie par confinement, les produits revendiqués augmentent le taux de survie jusqu'à 60% dans certains cas.

6) Activité anticonvulsivante

Les dérivés selon l'invention ne protègent pas la souris dans la crise convulsive provoquée au pentétrazole.

Quelques exemples non limitatifs de la présente demande sont décrits ci-après.

Exemple 1

N-(pyridyl-3 methyl)-cycloheptane carboxamide chlorhydrate

On coule en une heure à température ordinaire 0,1 mole de picolylamine en solution dans 30 ml de chloroforme, sur 0,11 mole de chlorure de l'acide cycloheptane carboxylique mis en solution dans 75 ml de chloroforme.

On maintient à température ordinaire sous agitation pendant 24 heures, filtre éventuellement un léger insoluble constitué par du chlorhydrate d'amine et évapore à sec. Le résidu repris à l'eau est traité par la soude aqueuse jusqu'à pH 10 et extrait au chloroforme.

Après lavages à l'eau de la couche organique et évaporation, le produit brut est recristallisé dans un mélange benzène/cyclohexane.

On obtient ainsi le dérivé du titre à l'état de base avec un rendement de 80% sous forme d'un solide blanc de pF 65/66°C présentant en chromatographie en couche mince sur silice un seul spot de Rf 0,95 dans le système A: acétate d'éthyle 2, isopropanol 2, ammoniaque concentrée 1 et de Rf = 0,60 dans le système B : benzène 80, méthanol 20.

Par traitement de la solution acétonique de cette base par l'acide chlorhydrique, on isole le chlorhydrate, dérivé du titre sous forme de cristaux blancs de pF 83/85°C.

En opérant dans les conditions de l'exemple 1 avec les chlorures d'acide correspondants, on obtient les dérivés de formule:

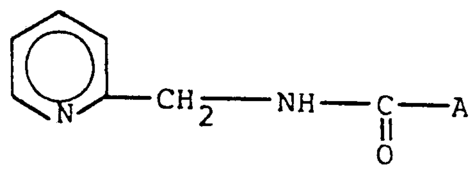

présentant les caractéristiques physico chimiques suivantes:

| A | Rdt | pF base | pF, HCl | Rf ds A | Rf ds B |
|---|---|---|---|---|---|
| Ex 2 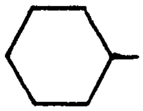 | 82 % | 98/99 | 79/80 | 0,95 | 0,50 |
| Ex 3 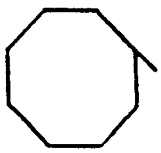 | 67 % | huile | 87 | 0,95 | 0,50 |
| Ex 4 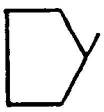 | 75 % | 71 | 117 | 0,95 | 0,50 |
| Ex 5 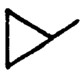 | 70 % | 70 | 178/79 | 0,95 | 0,55 |
| Ex 6 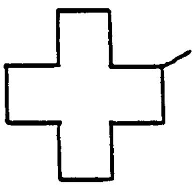 | 83 % | 129/130 | 181/183 | 0,95 | 0,59 |
| Ex 7 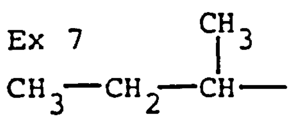 | 80 % | huile | 65 | 0,95 | 0,65 |
| Ex 8 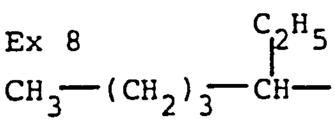 | 90 % | 35/36 | 78/80 | 0,95 | 0,67 |

En opérant dans les conditions de l'exemple 1 et en utilisant la pyridyl-2 méthanamine avec les chlorures d'acides appropriés, on obtient les dérivés de formule:

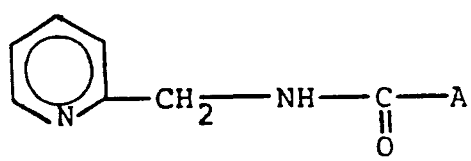

présentant les caractéristiques physico chimiques suivantes:

| A | Rdt | pF base | pF, HCl | Rf ds A | Rf ds B |
|---|---|---|---|---|---|
| Ex 9 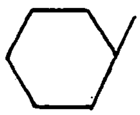 | 85 % | 95 | 135 | 0,90 | 0,60 |
| Ex 10 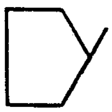 | 65 % | 65 | 130 | 0,90 | 0,60 |
| Ex 11 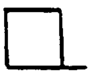 | 70 % | huile | 70 | 0,90 | 0,65 |
| Ex 12 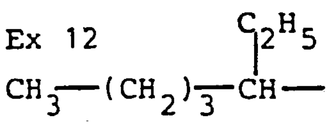 | 92 % | 40 | 85/87 | 0,90 | 0,70 |
| Ex 13 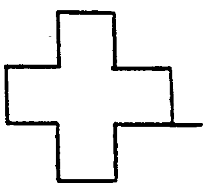 | 85 % | 136/138 | 190 | 0,90 | 0,65 |

En opérant dans les conditions de l'exemple 1 et en utilisant la pyridyl-4 méthanamine et les chlorures d'acide correspondants, on obtient les dérivés de formule générale:

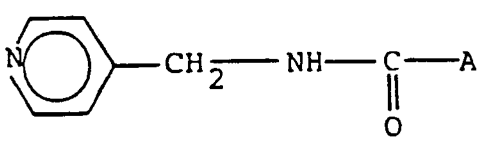

présentant les caractéristiques physico chimiques suivantes:

| A | Rdt | pF base | pF, HCl | Rf ds A | Rf ds B |
|---|---|---|---|---|---|
| Ex 14 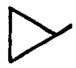 | 72 % | 60 | 135 | 0,92 | 0,50 |
| Ex 15 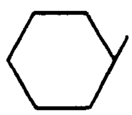 | 80 % | 80/85 | 100 | 0,92 | 0,50 |
| Ex 16 $C_2H_5$<br>$CH_3-(CH_2)_3-CH-$ | 85 % | huile | 85/90 | 0,92 | 0,60 |

Exemple 17

$N_1$ oxyde du N-(pyridyl-3 methyl) ethyl-2 hexanamide

On dissout 20 millimoles de N-(pyridyl-3 méthyl) éthyl-2 hexanamide base (exemple 7) dans 50 ml d'acide acétique puis ajoute 10 ml d'eau oxygénéeà 40%. On porte ensuite 3 heures à 80° puis évapore au bain marie sous vide la majeure partie de l'acide acétique, reprend à l'eau glacée et alcalinise à pH 10,5 par de la soude diluée.

On extrait au chloroforme, lave à l'eau et évapore le solvant.

Après reprise à l'éther, on obtient le dérivé du titre avec un rendement de 61% sous forme de cristaux blancs de pF 107/108°C présentant en CCM sur silice un seul spot de Rf = 0,25 dans le système B et de 0,45 dans le système : n butanol 8, acide acétique 1, eau 1. Le spectre IR enregistré dans KBr fait apparaître une bande N→O à 1290 $cm^{-1}$.

Exemple 18

$N_1$ oxyde du N-(pyridyl-3 methyl) cyclohexane carboxamide

En opérant dans les conditions de l'exemple précédent à partir du N-(pyridyl-3 méthyl) cyclohexane carboxamide base décrit à l'exemple 2, on obtient le dérivé du titre sous forme de cristaux blancs de pF 90/92°C, présentant un seul spot en CCM dans le système A de Rf = 0,30 et un seul spot de Rf 0,33 dans le système n butanol 8, eau 1, acide acétique 1. Le spectre IR enregistré dans le KBr fait apparaître une bande N→O à 1290 $cm^{-1}$.

**Revendications pour les Etats contractants: BE CH DE FR GB IT LI LU NL SE**

1. Composé de formule générale I ou un de ses sels pharmaceutiquement acceptables:

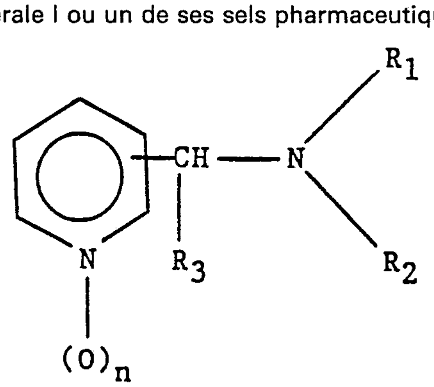

(I)

dans laquelle:

n est égal à 1 ou à 0,

$R_3$ représente un groupement alkyl de $C_1$ à $C_3$ ou un atome d'hydrogène,

$R_1$ représente un atome d'hydrogène,

$R_2$ représente un groupement:

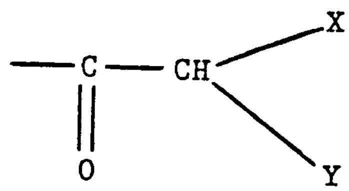

(II)

dans lequel:

X et Y sont différents,

X et Y représentent chacun un enchainement hydrocarboné saturé ou insaturé linéaire ou ramifié comprenant de 1 à 16 atomes de carbone,

X et Y déterminent ensemble un radical cyclique saturé ou insaturé comprenant de 3 à 12 atomes de carbone.

2. Un composé selon la revendication 1 dans lequel l'azote cyclique de la pyridine est à l'état de N oxyde.

3. N-(pyridyl-3 méthyl)-cyclooctane carboxamide
N-(pyridyl-3 méthyl)-cycloheptane carboxamide
N-(pyridyl-3 méthyl)-cyclohexane carboxamide
N-(pyridyl-3 méthyl)-cyclopentane carboxamide
N-(pyridyl-3 méthyl)-cyclopropane carboxamide
N-(pyridyl-3 méthyl)-éthyl-2 hexanamide
N-(pyridyl-3 méthyl)-cyclododecane carboxamide

4. N-(pyridyl-4 méthyl)-cyclopropane carboxamide
N-(pyridyl-4 méthyl)-cyclohexane carboxamide
N-(pyridyl-4 méthyl)-éthyl-2 hexanamide

5. N-(pyridyl-2 méthyl)-cyclododecane carboxamide
N-(pyridyl-2 méthyl)-cyclobutane carboxamide
N-(pyridyl-2 méthyl)-éthyl-2 hexanamide

6. $N_1$ oxyde du N-(pyridyl-3 méthyl)-cyclohexane carboxamide
$N_1$ oxyde du N-(pyridyl-3 méthyl)-éthyl-2 hexanamide

7. Procédé de préparation des dérivés selon les revendications 1—3—4—5 caractérisé en ce que l'on fait reàgir

un dérivé de formule générale III:

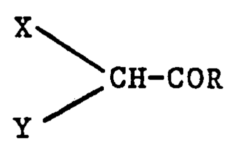

(III)

dans laquelle

X et Y sont tels que définis ci-dessus, et

R représente

un atome d'halogène,

un groupe

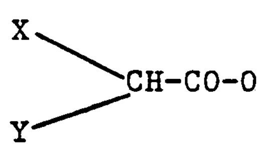

un groupe OH,

un groupe

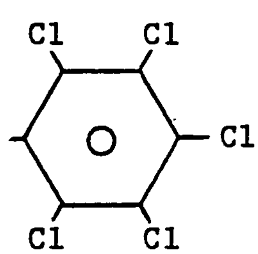

un groupe $(CH_3)_3C—CO—O$, ou
un groupe $C_2H_5—O—CO—O$, et

sur une pyridinealkylamine en quantité équimoléculaire, en solution dans un solvant inerte approprié, éventuellement en présence d'un accepteur d'hydrogène notamment une amine tertiaire choisie notamment dans le groupe qui comprend la triéthylamine, la pyridine, la picoline, à une température comprise entre la température ambiante et la température de reflux du milieu réactionnel.

8. Procédé selon la revendication 7 selon lequel le dérivé réactif de formule III est un halogénure d'acide qui est mis en réaction avec un équivalent moléculaire de pyridineméthanamine et en ce que la réaction est conduite dans le chloroforme à température ordinaire.

9. Procédé d'obtention des dérivés selon la revendication 2 où l'on traite les produits de formule générale I par l'eau oxygénée en solution dans l'acide acétique à une température comprise entre l'ambiance et l'ébullition.

10. Une composition pharmaceutique comprenant au moins un des dérivés selon les revendications 1 et 2 éventuellement converti en un sel pharmacologiquement acceptable.

11. Médicament à usage vétérinaire et/ou humain, constitué par au moins un des composés selon les Revendications 1 et 2.

**Revendications pour les Etats contractants: AT ES GR**

1. Procédé de préparation de dérivés de formule générale

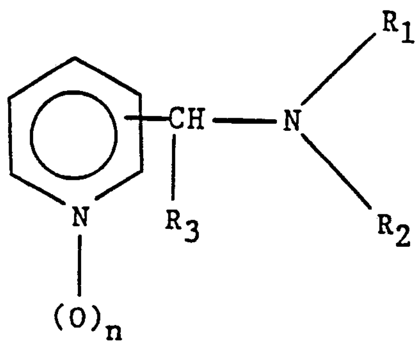

(I)

dans laquelle:

n est égal à 1 ou à 0,
$R_3$ représente un groupement alkyle de $C_1$ à $C_3$ ou un atome d'hydrogène,
$R_1$ représente un atome d'hydrogène,
$R_2$ représente un groupement

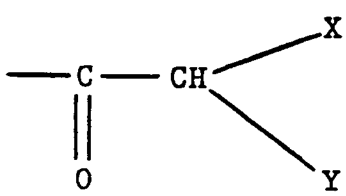

(II)

dans lequel:

X et Y sont différents

X et Y représentent chacun un enchainement hydrocarboné saturé ou insaturé linéaire ou ramifié comprenant de 1 à 16 atomes de carbone,

X et Y déterminent ensemble un radical cyclique saturé ou insaturé comprenant de 3 à 12 atomes de carbone, caractérisé en ce que l'on fait reàgir N-acylation entre

un dérivé de formule générale III:

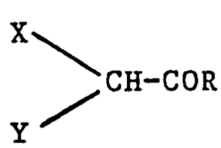

(III)

dans laquelle

X et Y sont tels que définis ci-dessus, et

R représente

un atome d'halogène,

un groupe

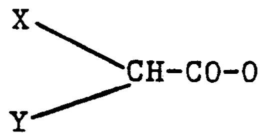

un groupe OH,

un groupe

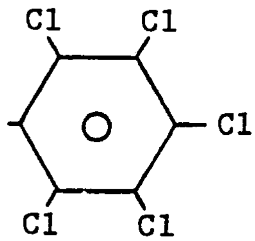

un groupe $(CH_3)_3C$—CO—O, ou

un groupe $C_2H_5$—O—CO—O, et

sur une pyridinealkylamine en quantité équimoléculaire, en solution dans un solvant inerte approprié, éventuellement en présence d'un accepteur d'hydrogène notamment une amine tertiaire choisie notamment dans le groupe qui comprend la triéthylamine, la pyridine, la picoline, à une température comprise entre la température ambiante et la température de reflux du milieu réactionnel.

2. Procédé selon la revendication 1, caractérisé en ce que le dérivé réactif de formule III est un halogénure d'acide qui est mis en réaction avec un équivalent moléculaire de pyridineméthanamine et en ce que la réaction est conduite dans le chloroforme à température ordinaire.

3. Procédé selon la revendication 1 ou la revendication 2, caractérisé en ce que l'azote cyclique de la pyridine est à l'état de N-oxyde.

4. Procédé selon l'une quelconque des revendications 1 à 3, caractérisé en ce que l'on traite les produits de formule générale I par l'eau oxygénée en solution dans l'acide acétique à une température comprise entre l'ambiance et l'ébullition.

**Patentansprüche für die Vertragsstaaten: BE CH DE FR GB IT LI LU NL SE**

1. Verbindung der allgemeinen Formel I

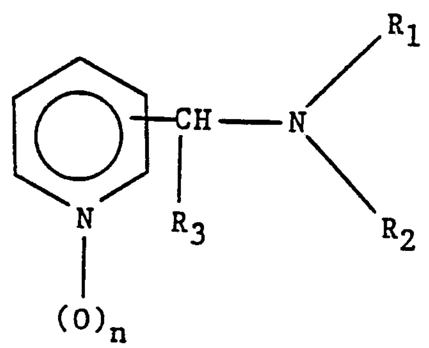

(I)

in der

n 1 oder 0 ist,

$R_3$ eine $C_1$- bis $C_3$-Alkylgruppe oder ein Wasserstoffatom bedeutet,

$R_1$ ein Wasserstoffatom bedeutet,

$R_2$ für eine Gruppe

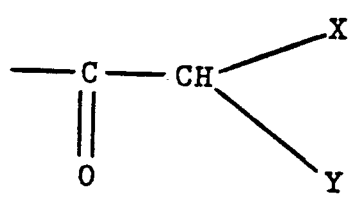

(II)

steht, in der

X und Y verschieden sind und

X und Y jeweils eine gesättigte oder ungesättigte, lineare oder verzweigte Kohlenwasserstoffkette mit 1 bis 16 Kohlenstoffatomen bedeuten oder

X und Y zusammen eine cyclische, gesättigte oder ungesättigte Gruppe mit 3 bis 12 Kohlenstoffatomen bilden, oder eines ihrer pharmazeutisch annehmbaren Salze.

2. Verbindung nach Anspruch 1, bei der das Ring-Stickstoffatom des Pyridins als N-Oxid vorliegt.

3. N-(3-Pyridylmethyl)-cyclooctan-carboxamid,
N-(3-Pyridylmethyl)-cycloheptan-carboxamid,
N-(3-Pyridylmethyl)-cyclohexan-carboxamid,
N-(3-Pyridylmethyl)-cyclopentan-carboxamid,
N-(3-Pyridylmethyl)-cyclopropan-carboxamid,
N-(3-Pyridylmethyl)-2-ethyl-hexanamid,
N-(3-Pyridylmethyl)-cyclododecan-carboxamid.

4. N-(4-Pyridylmethyl)-cyclopropan-carboxamid,
N-(4-Pyridylmethyl)-cyclohexan-carboxamid,
N-(4-Pyridylmethyl)-2-ethyl-hexanamid.

5. N-(2-Pyridylmethyl)-cyclododecan-carboxamid,
N-(2-Pyridylmethyl)-cyclobutan-carboxamid,
N-(2-Pyridylmethyl)-2-ethyl-hexanamid.

6. $N_1$-Oxid von N-(3-Pyridylmethyl)-cyclohexancarboxamid,
$N_1$-Oxid von N-(3-Pyridylmethyl)-2-ethylhexanamid.

7. Verfahren zur Herstellung der Verbindungen nach den Ansprüchen 1, 3, 4 und 5, dadurch gekennzeichnet, daß man eine Verbindung der allgemeinen Formel III

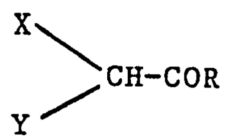

(III)

in der

X und Y wie oben definiert sind und

R für

ein Halogenatom,

eine Gruppe

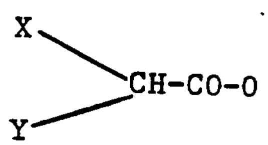

eine OH-Gruppe,

eine Gruppe

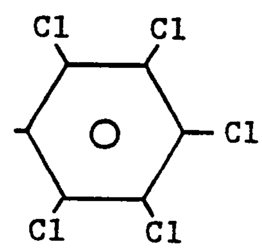

eine $(CH_3)_3C—COO$-Gruppe oder

eine $C_2H_5—O—COO$-Gruppe steht,

mit einem Pyridinalkylamin in äquimolarer Menge in Lösung in einem geeigneten inerten Lösungsmittel, gegebenenfalls in Gegenwart eines Wasserstoffakzeptors, vor allem eines tertiären Amins, ausgewählt vor allem aus der Gruppe, die Triethylamin, Pyridin und Pikolin umfaßt, bei einer Temperatur im Bereich zwischen Raumtemperatur und Rückflußtemperatur des Reaktionsmediums zur Reaktion bringt.

8. Verfahren nach Anspruch 7, dadurch gekennzeichnet, daß das reaktionsfähige Derivat der Formel III ein Säurehalogenid ist, das mit einem molaren Äquivalent Pyridinmethanamin umgesetzt wird, und daß die Umsetzung in Chloroform bei Raumtemperatur vorgenommen wird.

9. Verfahren zum Erhalt der Verbindungen nach Anspruch 2, dadurch gekennzeichnet, daß man die Verbindungen der allgemeinen Formel I mit Wasserstoffperoxid in Lösung in Essigsäure bei einer

Temperatur im Bereich von Raumtemperatur bis zur Siedetemperatur behandelt.

10. Pharmazeutisches Präparat, das mindestens eine der Verbindungen nach den Ansprüchen 1 und 2, gegebenenfalls in ein pharmakologisch annehmbares Salz überführt, enthält.

11. Arzneimittel zur Verwendung in der Tier- und/oder Humanmedizin, bestehend aus mindestens einer der Verbindungen nach den Ansprüchen 1 und 2.

**Patentansprüche für die Vertragsstaaten: AT ES GR**

1. Verfahren zur Herstellung von Verbindungen der allgemeinen Formel I

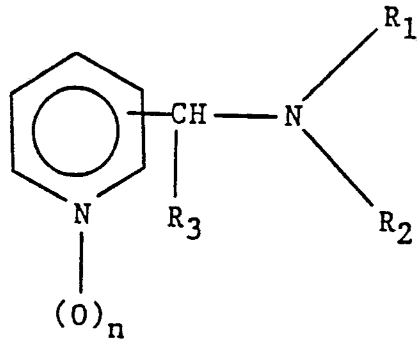

(I)

in der

n 1 oder 0 ist,

$R_3$ eine $C_1$- bis $C_3$-Alkylgruppe oder ein Wasserstoffatom bedeutet,

$R_1$ ein Wasserstoffatom bedeutet,

$R_2$ für eine Gruppe

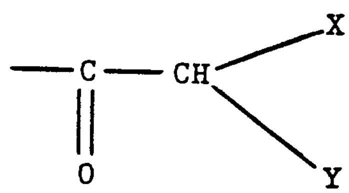

(II)

steht, in der

X und Y verschieden sind und

X und Y jeweils eine gesättigte oder ungesättigte, lineare oder verzweigte Kohlenwasserstoffkette mit 1 bis 16 Kohlenstoffatomen bedeuten oder

X und Y zusammen eine cyclische, gesättigte oder ungesättigte Gruppe mit 3 bis 12 Kohlenstoffatomen bilden, dadurch gekennzeichnet, daß man eine Verbindung der allgemeinen Formel III

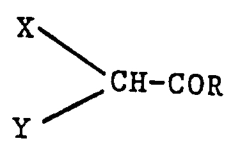

(III)

in der

X und Y wie oben definiert sind und

R für

ein Halogenatom,

eine Gruppe

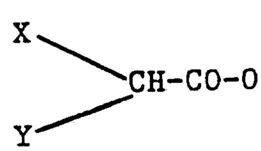

eine OH-Gruppe,
eine Gruppe

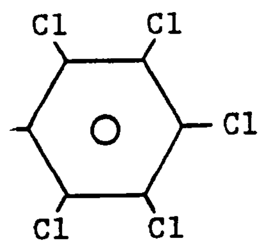

eine $(CH_3)_3C—COO$-Gruppe oder
eine $C_2H_5—O—COO$-Gruppe steht,

mit einem Pyridinalkylamin in äquimolarer Menge in Lösung in einem geeigneten inerten Lösungsmittel, gegebenenfalls in Gegenwart eines Wasserstoffakzeptors, vor allem eines tertiären Amins, ausgewählt vor allem aus der Gruppe, die Triethylamin, Pyridin und Pikolin umfaßt, bei einer Temperatur im Bereich zwischen Raumtemperatur und Rückflußtemperatur des Reaktionsmediums zur Reaktion bringt.

2. Verfahren nach Anspruch 1, dadurch gekennzeichnet, daß das reaktionsfähige Derivat der Formel III ein Säurehalogenid ist, das mit einem molaren Äquivalent Pyridinmethanamin umgesetzt wird, und daß die Umsetzung in Chloroform bei Raumtemperatur vorgenommen wird.

3. Verfahren nach Anspruch 1 oder 2, dadurch gekennzeichnet, daß der Ring-Stickstoff des Pyridins als N-Oxid vorliegt.

4. Verfahren nach einem der Ansprüche 1 bis 3, dadurch gekennzeichnet, daß man die Verbindungen der allgemeinen Formel I mit Wasserstoffperoxid in Lösung in Essigsäure bei einer Temperatur im Bereich von Raumtemperatur bis zur Siedetemperatur behandelt.

**Claims for the Contracting States: BE CH DE FR GB IT LI LU NL SE**

1. Compound of the general formula I or one of its pharmaceutically acceptable salts:

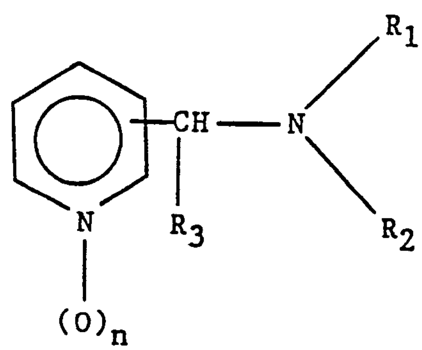

(I)

in which:
n is equal to 1 or to 0,
$R_3$ represents an alkyl group of $C_1$ to $C_3$ or a hydrogen atom,
$R_1$ represents a hydrogen atom,
$R_2$ represents a group

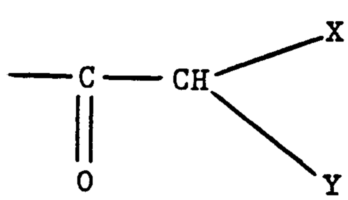

(II)

in which:
X and Y are different
X and Y each represent a linear or branched, saturated or unsaturated, hydrocarbon chain comprising from 1 to 16 carbon atoms,
X and Y determine together a saturated or unsaturated cyclic radical comprising from 3 to 12 carbon atoms.

2. A compound according to claim 1 in which the ring nitrogen of the pyridine is in the state of N oxide.

3. N-(pyridyl-3 methyl)-cyclooctane carboxamide
N-(pyridyl-3 methyl)-cycloheptane carboxamide
N-(pyridyl-3 methyl)-cyclohexane carboxamide
N-(pyridyl-3 methyl)-cyclopentane carboxamide
N-(pyridyl-3 methyl)-cyclopropane carboxamide
N-(pyridyl-3 methyl)-ethyl-2 hexanamide
N-(pyridyl-3 methyl)-cyclododecane carboxamide

4. N-(pyridyl-4 methyl)-cyclopropane carboxamide
N-(pyridyl-4 methyl)-cyclohexane carboxamide
N-(pyridyl-4 methyl)-ethyl-2 hexanamide

5. N-(pyridyl-2 methyl)-cyclododecane carboxamide
N-(pyridyl-2 methyl)-cyclobutane carboxamide
N-(pyridyl-2 methyl)-ethyl-2 hexanamide

6. $N_1$ oxide of N-(pyridyl-3 methyl)-cyclohexane carboxamide
$N_1$ oxide of N-(pyridyl-3 methyl)-ethyl-2 hexanamide

7. Process for the preparation of derivatives according to claims 1, 3, 4, 5 characterized in that one reacts:

a derivative of the general formula III:

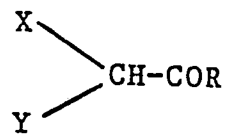

(III)

in which

X and Y are as defined above, and

R represents:

a halogen atom,

an 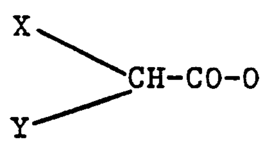 group,

an OH group,

a 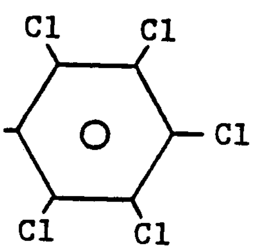 group,

a $(CH_3)_3C—CO—O$ group, or

a $C_2H_5—O—CO—O$ group, and

on a pyridinealkylamine in equimolecular amount, in solution in a suitable inert solvent, if necessary in the presence of a hydrogen acceptor, particularly a tertiary amine selected particularly from the group which comprises triethylamine, pyridine, picoline, at a temperature comprised between room temperature and the reflux temperature of the reaction medium.

8. Process according to claim 7 in which the reagent derivative of formula III is an acid halide which is reacted with a molecular equivalent of pyridinemethanamine and wherein the reaction is conducted in chloroform at ordinary temperature.

9. Process for obtaining derivatives according to claim 2 wherein the products of general formula I are treated with hydrogen peroxide in solution in acetic acid at a temperature comprised between room temperature and boiling point.

10. A pharmaceutical composition comprising at least one of the derivatives according to claims 1 and 2 possibly converted into a pharmacologically acceptable salt.

11. Medicament for veterinary and/or human use, constituted by at least one of the compounds according to claims 1 and 2.

**Claims for the Contracting States: AT ES GR**

1. Process for the preparation of derivatives of the general formula

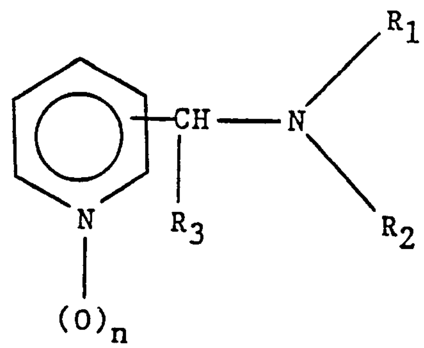

(I)

in which:

n is equal to 1 or to 0,
$R_3$ represents an alkyl group of $C_1$ to $C_3$ or a hydrogen atom,
$R_1$ represents a hydrogen atom,
$R_2$ represents a

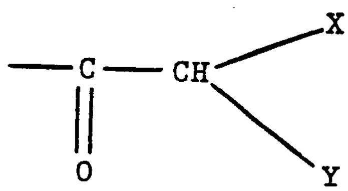

(II)

group in which:

X and Y are different

X and Y each represent a linear or branched, saturated or unsaturated, hydrocarbon chain comprising from 1 to 16 carbon atoms,

X and Y determine together a saturated or unsaturated cyclic radical comprising from 3 to 12 carbon atoms, characterized in that one reacts:

a derivative of the general formula III:

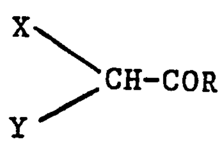

(III)

in which

X and Y are as defined above, and
R represents:
a halogen atom,

an 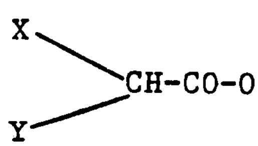 group,

an OH group,

a 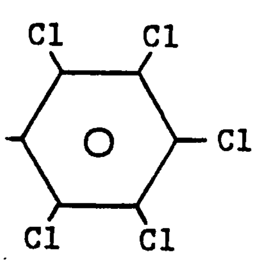 group

a $(CH_3)_3C—CO—O$ group, or

a $C_2H_5—O—CO—O$ group, and

on a pyridinealkylamine in equimolecular amount, in solution in a suitable inert solvent, if necessary in the presence of a hydrogen acceptor, particularly a tertiary amine selected particularly from the group which comprises triethylamine, pyridine, picoline, at a temperature comprised between room temperature and the reflux temperature of the reaction medium.

2. Process according to claim 1, characterized in that the reagent derivative of formula III is an acid halide which is reacted with the molecular equivalent of pyridinemethanamine and wherein the reaction is conducted in chloroform at ordinary temperature.

3. Process according to claim 1 or claim 2, characterized in that the ring nitrogen of the pyridine is in the state of N-oxide.

4. Process according to any one of claims 1 to 3, characterized in that the products of general formula I are treated with hydrogen peroxide in solution in acetic acid at a temperature comprised between room temperature and boiling point.